(19) Europäisches Patentamt
European Patent Office
Office européen des brevets

(11) Veröffentlichungsnummer: **0 273 439**
**A2**

# (12) EUROPÄISCHE PATENTANMELDUNG

(21) Anmeldenummer: **87119290.2**

(22) Anmeldetag: **29.12.87**

(51) Int. Cl.⁴: **C07C 103/46**, C07K 7/00, A61K 37/02

(30) Priorität: **05.01.87 DE 3700173**

(43) Veröffentlichungstag der Anmeldung:
**06.07.88 Patentblatt 88/27**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Günther, Jung, Prof.Dr.**
**Ob der Grafenhalde 5**
**D-7400 Tübingen(DE)**
Erfinder: **Metzger, Jörg**
**Ebertstrasse 5**
**D-7400 Tübingen(DE)**

## (54) Verfahren zur Herstellung von lipophilen Aminosäurederivaten sowie lipophile Aminosäurederivate.

(57) Lipophile Aminosäurederivate der allgemeinen Formel

$$R - CH_2 - CO - \underset{R}{\underset{|}{CH^*}} - CO - NH - \underset{R'}{\underset{|}{CH^*}} \cdot COX \qquad (II)$$

in der R, R′ und X die angegebene Bedeutung haben, werden hergestellt, indem ein Aldoketen-Dimers der allgemeinen Formel

$$\begin{array}{l} R - CH = C - CH - R \\ \phantom{R - CH = {}} | \quad\;\; | \\ \phantom{R - CH = {}} O - C = O \end{array} \qquad (I)$$

mit einem Aminosäureester mit freiem N-Terminus umgesetzt werden. Die Verbindungen der Formel II und weitere lipophile Aminosäurederivate, die aus den Verbindungen der Formel II hergestellt werden, eignen sich zur Anwendung als Adjuvans.

EP 0 273 439 A2

## Verfahren zur Herstellung von lipophilen Aminosäurederivaten sowie lipophile Aminosäurederivate

Die Erfindung betrifft Verfahren zur Herstellung von lipophilen Aminosäurederivaten sowie danach erhältliche lipophile Aminosäurederivate.

Lipophile Derivate von Aminosäuren und Peptiden, die hier unter Aminosäurederivaten verstanden werden sollen, besitzen unter anderem als Membranankerverbindungen, wie sie beispielsweise in der DE-OS 35 46 150.0 beschrieben sind, steigende Bedeutung.

Sie eignen sich beispielsweise als Adjuvans bei der Antikörperherstellung und sind B-Zell-Mitogene, wie durch $^3$H-Thymidin-Einbau gezeigt werden konnte. Dabei haben sich insbesondere Aminosäureverbindungen mit α-Alkyl-β-hydroxyacylresten (Mycolsäurereste), deren Hydroxyfunktion mit einer Fettsäure verestert ist, als lipophilem Anteil bewährt. Mycolsäuren sind in natürlichen Lipoiden enthalten und, wie Muramyldipeptid, effiziente Komponenten von Freunds Adjuvans, einer zur Antikörperbildungs-Induktion eingesetzte Mischung von abgetöteten Tuberkel-Bazillen in Paraffinöl. Einfache Mycoloylaminosäuren wie z.B. Mycoloyl-D-alanin oder Mycoloyl-D-glutaminsäure besitzen jedoch keine Adjuvansaktivität (A. Tanaka, K. Tanaka, T. Tsubone, Y. Kuroda, K. Sugiyama, Int. Arch. Allergy 28, 340 - 52 (1965)). O-acylierte β-Hydroxyacylreste finden sich auch in Lipid A, dem endotoxischen Prinzip des Endotoxins gramnegativer Bakterien.

Es ist außerdem bekannt (Adjuvant properties of hydrophobic derivatives prepared from L-tyrosine; A.W. Wheeler, N. Whittall, V. Spackman, D.M. Moran: Int. Arch. Allergy App. Immunol., 1984, 75(4), 294-9), daß N-und O-Acyl-Derivate von Tyrosin die Antikörper-Produktion gegen Graspollen im System Meerschweinchen induzieren und zwar um so stärker, je länger die hier verwendete Acylkette ist.

Bisher wurden beispielsweise Mycoloyl-Peptide über die Acylierung mit Mycolsäuren (Fractionation and characterization of wax D, a peptidoglycolipid of Mycobacterium tuberculosis, A. Tanaka, K. Tanaka, T. Tsubone, Y. Kuroda, K. Sugiyama, Int. Arch. Allergy 28, 340-52 (1965); Synthesis and biological activities of mycoloylamino acids, Y. Yamamura, A. Tanaka, M. Kato, J. Biochem. 47, 505-512 (1960)) aufwendig hergestellt, wobei die Mycolsäuren selbst ihrerseits erst durch Claisen-Esterkondensation, Reduktion und anschließende Verseifung synthetisiert werden müssen oder aus Bakterienzellwänden isoliert werden müssen.

Es ist demzufolge Aufgabe der Erfindung, ein einfacheres Verfahren zur Herstellung von lipophilen Derivaten von Aminosäuren sowie neue Lipopeptide mit höherer Lipophilie zu schaffen.

Die Aufgabe wird erfindungsgemäße durch Umsetzen eines Aldoketen-Dimeren der allgemeinen Formel (I)

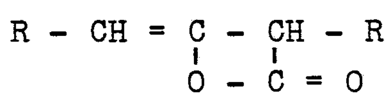

(I)

wobei R = Alkyl mit mehr als 6 C;
mit einem Aminosäureester mit freiem N-Terminus zu einem α-Alkyl-β-ketoacyl-aminosäurederivat der allgemeinen Formel (II):

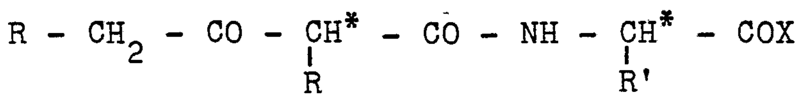

(II)

wobei R′ = Seitenkette einer (Protein)-Aminosäure oder H und X = Schutzgruppe für eine Carboxylsäuregruppe, wie OBu$^t$, OMe, OBzl oder dergleichen oder eine geschützte Aminosäure oder Peptid ist, gelöst. Die Konfiguration an den beiden asymmetrischen C-Atomen kann dabei jeweils R oder S sein.

Weiterhin betrifft die Erfindung neue lipophile Aminosäureverbindungen der allgemeinen Formel (V):

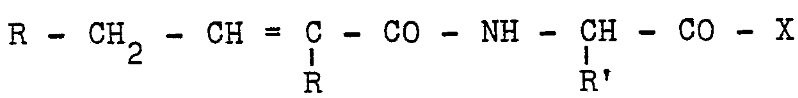

(V)

wobei R = Alkyl mit mehr als 6 C; R′ = Seitenkette einer L-oder D-(Protein)-Aminosäure oder H ist und X = Schutzgruppe für Carboxylsäuregruppe, eine geschützte Aminosäure oder Peptid oder -OH ist und

lipophile Aminosäureverbindungen der allgemeinen Formel (IV):

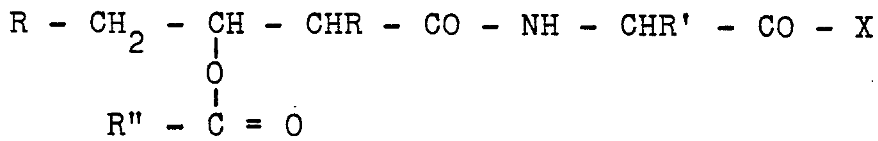 (IV)

wobei R = Alkyl mit mehr als 6 C; R' = Seitenkette einer L-oder D-(Protein)-Aminosäure oder H ist und X = Schutzgruppe für Carboxylsäuregruppe, eine geschützte Aminosäure oder Peptid oder -OH ist.

Die nach dem erfindungsgemäßen Verfahren über α-Alkyl-β-keto-acyl-aminosäureverbindungen hergestellten Lipopeptide sind lipophiler als einfache Fettsäure-Derivatverbindungen; beispielsweise zeigen sie starke mitogene Aktivität gegenüber Milzzellen der Maus und sollten sich deshalb auch besser als Adjuvans eignen als einfache Fettsäurederivate.

Vorteilhafterweise ist dabei das Aldoketen-Dimere (I) ein Fettsäure-Aldoketen-Dimeres, wie es beispielsweise leicht aus den entsprechenden Säurehalogeniden mit tertiären Basen zugänglich ist (s. "Ketene dimers from acid halides, HJ.C. Sauer, J. Am. Chem. Soc. 69, 2448 (1947); Preparation of ketene dimers derived from long-chained aliphatic acids, S. Piekarski, Comp. rend. 238, 1241-43 (1954); Ketene dimers from normal, long-chain aliphatic acids, S. Piekarski, J. recherches centre natl. recherche sci. labs. Bellevue, 40, 197-230 (1958), DE-OS 23 35 488 und DE-OS 29 27 118).

Bevorzugt wird das Aldoketen-Dimere mit dem Aminosäureester in Gegenwart einer katalytischen Menge eines Dialkylaminopyridins mit Niederalkylresten ($C_1$-$C_6$), bevorzugt von Dimethylaminopyridin umgesetzt.

Bei einer besonders bevorzugten Ausführungsform des erfindungsgemäßen Verfahrens findet die Umsetzung in einem organischen Lösungsmittel, bevorzugt trockenem Pyridin, statt.

Die Umsetzung erfolgt vorteilhafterweise bei einer Temperatur zwischen 0 und 100°C, bevorzugt zwischen 25 und 80°C und ganz besonders bevorzugt zwischen 40 und 60°C.

Durch Reduktion der N-α-Alkyl-β-ketoacyl-Verbindung (II), bevorzugt mit einem Borhydrid in an sich bekannter Weise, können N-α-Alkyl-β-hydroxy-acyl-Verbindungen der allgemeinen Formel (III):

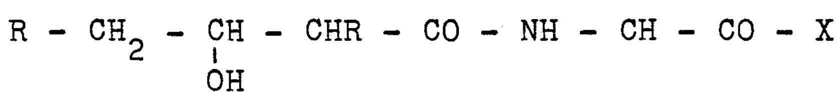 (III)

erhalten werden, wobei auch hier X für eine Aminosäure bzw. ein Peptid steht.

Diese Verbindungen können zur Erhöhung der Lipophilie durch Veresterung des Reaktionsproduktes (III) in an sich bekannter Weise, bevorzugt durch Umsetzung mit einem Säureanhydrid, zu Estern der nachfolgenden allgemeinen Formel (IV):

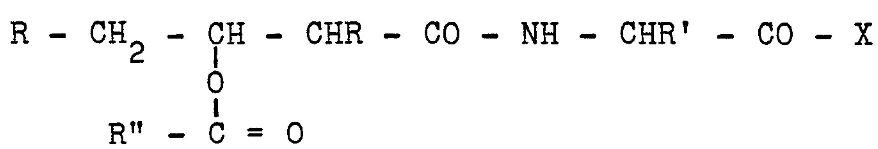 (IV)

umgesetzt werden, wobei auch hier als Säuren Fettsäuren besonders bevorzugt sind.

Die Abspaltung der Carbonsäurefunktions-Schutzgruppe, bevorzugt mit $CF_3COOH$ unter Erhalt der freien Carbonsäurefunktion(en) liefert die Derivate der freien Aminosäuren bzw. Peptide wie sie für viele biologische/medizinische Anwendungen erwünscht sind.

Erfindungsgemäß kann die α-Alkyl-β-hydroxy-Verbindung mit Acetanhydrid und $KHSO_4$ unter Erhalt von Verbindungen der allgemeinen Formel (V):

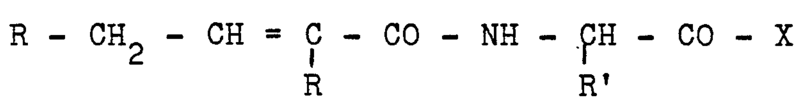 (V)

umgesetzt werden, wobei R = Alkyl mit mehr als 6 C; R' = Seitenkette einer L-oder D-(Protein)-Aminosäure oder H und X = Schutzgruppe für Carboxylsäuregruppe, eine geschützte Aminosäure oder Peptid oder -OH ist.

Die Umsetzung eines höheren dimeren Aldoketens, dem bekanntlich die Struktur eines 4-Alkyliden-3-alkyl-2-oxetanons zukommt:

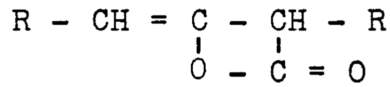

mit primären Aminen, Anilinderivaten, Alkylaminen ist schon seit längerem bekannt und liefert $\beta$-Ketoamine mit einer Alkylkette in $\alpha$-Stellung:

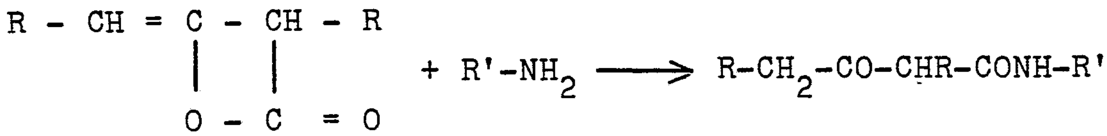

Eine in der Literatur noch nicht beschriebene Anwendung dieser Reaktion ist die Umsetzung von langkettigen Diketenen mit Aminosäurederivaten, z.B. Aminosäureestern oder geschützten Peptiden mit freiem N-Terminus. Man erhält auf diese Weise lipophile Aminosäure-bzw. Peptid-Derivate der allgemeinen Formel:

$$R - CH_2 - CO - \underset{R}{CH^*} - CO - NH - \underset{R'}{CH^*} - CO - X$$

mit R = Alkyl (länger als 6 C-Atome), R′ = Seitenkette einer (Protein-)Aminosäure oder Wasserstoff und X = Alkoxy-Gruppe, wie OBu$^t$, OMe, OBzl oder eine geschützte Aminosäure bzw. eine Aminosäuresequenz.

Der $\alpha$-Alkyl-$\beta$-keto-acyl-Rest:

$$R - CH_2 - CO - \underset{R}{CH^*} - CO -$$

sowie die N-terminale Amidbindung sind gegenüber $CF_3COOH$ stabil, so daß säurelabile Schutzgruppen wie Bu$^t$, Boc an der Aminosäure bzw. Peptid nach Einführung des $\alpha$-Alkyl-$\beta$-ketoacyl-Restes abgespalten werden können.

Die Verbindungen können sehr einfach mit $NaBH_4$ zu $\alpha$-Alkyl-$\beta$-hydroxy-Derivaten der allgemeinen Formel:

$$R - CH_2 - \underset{OH}{CH^*} - \underset{R}{CH^*} - CO - NH - CHR' - CO - X$$

mit R, R′ und X wie oben angegeben, reduziert werden. Diese Verbindungen stellen sekundäre Amide von Mycolsäuren dar. Beispielsweise liefert die Umsetzung des dimeren Tetradecylketens mit einem Aminosäureester nach der Reduktion ein Derivat der Corynomycolsäure, einer in den Lipiden von Corynebacterium diphtheriae vorkommenden Fettsäure.

Die Reduktion kann vor oder nach Abspaltung der Schutzgruppe vorgenommen werden und liefert in beiden Fällen fast quantitative Ausbeuten. Die Verbindungen (II), (III) und (IV) besitzen ein bzw. zwei Asymmetriezentren im lipophilen Acylteil. Man erhält deshalb stets ein Diastereomeren-Gemisch, das sich beispielsweise an Kieselgel auftrennen läßt. Für den Einsatz als Membranankerverbindungen läßt sich jedoch auch das Diastereomerengemisch oder jeweiligen Verbindungen verwenden.

Die OH-Funktion in (III) läßt sich mit Fettsäureanhydriden in Gegenwart von Dimethylaminopyridin verestern, wobei man dreikettige Verbindungen der allgemeinen Formeln (IV)

$$R - CH_2 - \underset{\substack{| \\ O \\ | \\ R'' - C = O}}{CH} - CHR - CO - NH - CHR' - CO - X \qquad (IV)$$

erhält, wobei R″ einen beliebigen Alkylrest darstellt. Auch die Verbindungen (IV) sind gegenüber $CF_3COOH$ stabil, so daß sich auch hier leicht die ungeschützten Derivate herstellen lassen.

Die Umsetzung von höheren Aldoketen-Dimeren mit Aminosäure-Derivaten in Gegenwart einer tertiären Amino-Base, wie Triethylamin oder N-Methyl-morpholin in organischen Lösungsmitteln, wie Methylenchlorid, Chloroform oder Dimethylformamid verläuft normalerweise langsam und unvollständig, auch bei höheren Temperaturen. Unter diesen Bedingungen ist außerdem die stets auftretende Bildung eines "einfach" acylierten Aminosäure-Derivates, die formal einer Acylierung mit dem monomeren Aldoketen entspricht, die Hauptreaktion. Man erhält also z.B. bei der Reaktion eines Aminosäureesters mit dem Tetradecylketen-Dimeren hauptsächlich den N-Palmitoylaminosäureester.

Man erhält selbst bei 20-stündiger Reaktion bei 50°C nur etwa 20 % der gewünschten α-Alkyl-β-keto-acylverbindung; ein großer Teil des Diketens setzt sich nicht um. Führt man diese Reaktion in trockenem Pyridin bei Raumtemperatur durch, so läßt sich schon nach 3 Stunden kein Diketen mehr nachweisen. Die Bildung der "einfachen" Acylverbindung wird unter diesen Bedingungen zur Nebenreaktion. Eine weitere Verkürzung der Reaktionszeit läßt sich durch Erwärmung des Reaktionsansatzes und auch durch Zugabe von katalytischen Mengen an Dimethylaminopyridin erreichen.

Die Umsetzung von höheren Aldoketen-Dimeren mit ~~Aminosäure-und Peptid-~~Derivaten ist also ein sehr einfacher und billiger Weg zur Darstellung von Lipopeptiden. Diese Verbindungen lassen sich in hohen Ausbeuten und kurzen Reaktionszeiten durch $NaBH_4$ zu Mycoloyl-Peptiden reduzieren.

Im Falle der Darstellung von Mycoloyl-Peptiden über Diketene wird nach beendeter Reduktion der Reaktionsansatz einfach mit wäßriger Salzsäure angesäuert und das dadurch ausfallende, schon sehr reine Produkt abgesaugt. Ein Vorteil liegt auch darin, daß die Reduktion erst am Ende der Gesamtsynthese eines Lipopeptides erfolgen kann.

Über die Mycoloyl-Peptide lassen sich ihrerseits durch O-Acylierung neuartige dreikettige Verbindungen herstellen, die aufgrund ihrer höheren Lipophilie und einer stärkeren B-Zell-Mitogenität (s. Beisp. 17 und Fig. 1) oft den 2-kettigen Lipoaminosäuren vorzuziehen sind. Durch Eliminierung der OH-Gruppe lassen sich ungesättigte Verbindungen herstellen, die ebenfalls als Lipopeptide für verschiedene Anwendungszwecke brauchbar sind.

Nachfolgend wird die Herstellung einiger erfindungsgemäßer Verbindungen beschrieben:

**Beispiel 1**

**α-Hexadecyl-β-keto-arachidoyl-Gly-tert.-butylester**

1 mMol Stearinsäurediketen wird unter Rühren zu 1 mMol Gly-tert.-butylester in 3 ml wasserfreiem Pyridin gegeben; bei 50°C wird anschließend 0.1 mMol Dimethylaminopyridin zugegeben. Nach Einsetzen der Reaktion wechselt die Farbe der Lösung von gelb nach cognacfarben; nach 2 Stunden ist dünnschichtchromatographisch kein Diketen mehr nachweisbar. Das Pyridin wird im Vakuum verdampft und das Rohprodukt aus wenig Chloroform mit Methanol in der Kälte umgefällt. Mögliche Verunreinigungen durch überschüssiges Diketen lassen sich aus Essigester oder Diisopropylether bei 0°C ausfällen, wobei das oft noch gelbe bis orangefarbene Produkt in Lösung bleibt. Zur Endreinigung kann an einer Kieselgel-Säule mit Chloroform flashchromatographiert werden.

Die Ausbeute betrug 72 %.

Der $R_f$-Wert des Produktes in $CHCl_3$ auf Kieselgel-Platten 60 $F_{254}$, 10x20 (erhältlich von Merck, Darmstadt), Detektion mit Iod, betrug 0,47; in Ethylether als Laufmittel auf gleichen Platten 0,81. In Tabelle 1 sind die physikalischen Daten der Produkte weiterer Reaktionen, die unter den gleichen Bedingungen wie in Beispiel 1 durchgeführt wurden, angegeben.

<u>Tabelle I</u>

α-Hexadecyl-ß-keto-arachidoyl-aminosäure-t-butylester

| Beisp. Nr. | Aminosäureester (2) | Reaktionszeit [ h ] | Ausbeute % | Summenformel | Molmasse | Eigenschaften von (3) $R_f$-Wert[2] in CHCl$_3$ | EE | berechnet (%) C | H | N | gefunden (%) C | H | N |
|---|---|---|---|---|---|---|---|---|---|---|---|---|---|
| 1 | H-Gly-OBu$^t$.HCl | 2 | 72 | $C_{42}H_{81}NO_4$ | 664,11 | o,47 | o,81 | 75,96 | 12,29 | 2,11 | 75,85 | 12,41 | 1,9o |
| 2 | H-Ala-OBu$^t$.HCl | 3 | 76 | $C_{43}H_{83}NO_4$ | 678,13 | o,53 | o,81 | 76,22 | 12,35 | 2,o7 | 76,25 | 12,47 | 2,o1 |
| 3 | H-Ser(Bu$^t$)-OBu$^t$ | 3 | 52 | $C_{47}H_{91}NO_5$ | 75o,24 | o,46 | o,8o | 75,24 | 12,23 | 1,87 | 75,o1 | 12,41 | 1,75 |
| 4 | H-Lys(Boc)-OBu$^t$.HCl | 2 | 63 | $C_{51}H_{98}N_2O_6$ | 835,35 | o,14 | o,8o | 73,33 | 11,82 | 3,35 | 73,6o | 11,54 | 3,o7 |
| 5 | (H-Cys-OBu$^t$)$_2$ | 3 | 5o | $C_{86}H_{164}N_2O_8S_2$ | 1418,38 | o,41 | o,83 | 72,83 | 11,65 | 1,98 | 72,63 | 11,47 | 1,79 |
| 6 | H-Asp(Bu$^t$)-OBu$^t$.HCl | 3 | 62 | $C_{48}H_{91}NO_6$ | 778,25 | o,44 | o,79 | 74,o8 | 11,79 | 1,8o | 74,48 | 11,55 | 1,68 |
| 7 | H-Tyr(Bu$^t$)-OBu$^t$.HCl | 4 | 51 | $C_{53}H_{95}NO_5$ | 826,34 | o,45 | o,8o | 77,o4 | 11,59 | 1,7o | 77,51 | 11,78 | 1,65 |
| 8 | H-Phe-OBu$^t$.HCl | 3 | 62 | $C_{49}H_{87}NO_4$ | 754,23 | o,57 | o,82 | 78,o3 | 11,63 | 1,86 | 77,88 | 11,61 | 1,8o |

[2] Kieselgel.Platten 60 $F_{254}$ 10x20, Merck, 1 x vorequilibriert in EE bzw. $CHCl_3$; Detektion mit Jod.

## Beispiel 9

**α-Hexadecyl-β-hydroxy-arachidoyl-Gly-tert.-butylester**

1 mMol des α-Hexadecyl-β-keto-arachidoyl-Gly-butylester wird in 15 ml Propanol in der Wärme gelöst und mit festem Natriumborhydrid (1 mMol) versetzt. Das Reduktionsprodukt fällt allmählich aus, nach 3 Stunden Rühren wird die Fällung durch Neutralisation mit 0.1N HCl vervollständigt. Die farblose Mycoloyl-Verbindung wird abgesaugt, mehrfach mit Wasser gewaschen und über $P_2O_5$ getrocknet. Die physikalischen Werte der Verbindung sind in Tabelle II aufgeführt.

Nachfolgend werden tabellarisch die Ergebnisse weiterer Umsetzungen, die unter den gleichen Bedingungen wie in Beispiel 9 beschrieben, durchgeführt wurden, angegeben:

## Tabelle II

α-Hexadecyl-β-hydroxy-arachidoyl-aminosäure-t-butylester

| Beisp. Nr. | Aminosäure-Rest in 3 (Tab.1) | Ausbeute [ % ] | Summenformel | Molmasse | Schmp.[1] [°C] | Eigenschaften von 4: $R_f$-Wert[2] in CHCl$_3$ | EE | berechnet (%) C | H | N | gefunden (%) C | H | N |
|---|---|---|---|---|---|---|---|---|---|---|---|---|---|
| 9 | Gly-OBu$^t$ | 92 | $C_{42}H_{83}NO_4$ | 666,12 | 91 | o,19/o,28 | o,82 | 75,73 | 12,56 | 2,1o | 75,78 | 12,74 | 1,89 |
| 10 | Ala-OBu$^t$ | 92 | $C_{43}H_{85}NO_4$ | 68o,15 | 84 | o,17-o,24/ o,29-o,35 | o,81 | 75,94 | 12,6o | 2,o6 | 75,81 | 12,67 | 1,99 |
| 11 | Ser(Bu$^t$)-OBu$^t$ | 72 | $C_{47}H_{93}NO_5$ | 752,26 | 55 | o,21-o,26/ o,29-o,35 | o,81 | 75,o4 | 12,46 | 1,86 | 74,82 | 12,61 | 1,64 |
| 12 | Lys(Boc)-OBu$^t$ | 75 | $C_{51}H_{100}N_2O_6$ | 837,36 | 76 | o,o5/o,o7 | o,81 | 73,15 | 12,o4 | 3,35 | 72,93 | 11,83 | 3,31 |
| 13 | (Cys-OBu$^t$)$_2$ | 91 | $C_{86}H_{166}N_2O_8S_2$ | 142o,39 | 75 | o,o9-o,24 | o,82-o,87 | 72,72 | 11,78 | 1,97 | 72,54 | 11,76 | 1,89 |
| 14 | Asp(Bu$^t$)-OBu$^t$ | 74 | $C_{48}H_{93}NO_6$ | 78o,27 | 65 | 0,22-o,32 | o,81 | 73,89 | 12,o1 | 1,8o | 73,68 | 11,86 | 1,66 |
| 15 | Tyr(Bu$^t$)-OBu$^t$ | 92 | $C_{53}H_{97}NO_5$ | 828,35 | 78 | o,2o-o,27 | o,82 | 76,85 | 11,8o | 1,69 | 76,57 | 11,58 | 1,57 |
| 16 | Phe-OBu$^t$ | 77 | $C_{49}H_{89}NO_4$ | 756,25 | 75 | o,33-o,4o | o,81 | 77,82 | 11,86 | 1,85 | 77,53 | 11,58 | 1,56 |

[1] Schmelzpunkt d. Diastereomerengemisches, unkorrigiert

[2] Kieselgel-Platten 60 $F_{254}$, 10x20 cm, Merck, 1 x vorequilibriert in EE bzw. $CHCl_3$;
Detektion mit Jod. Die Diastereomerengemische in Tab. II ergeben 1 bis 2 langezogene Flecken, deren oberer und unterer Rand angegeben sind.

Durch folgendes Formelschema kann die Herstellung der Verbindungen gemäß den vorstehenden Beispielen verdeutlicht werden:

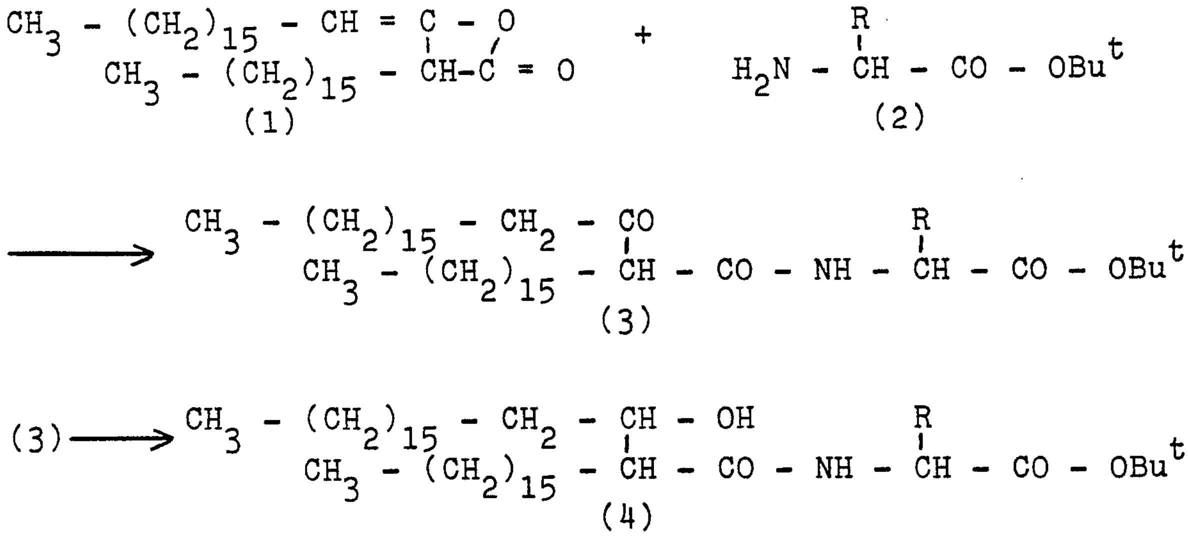

(1) (2) (3) (4)

Der erste Schritt der Reaktion kann in Pyridin mit ca. 10 % DMAP bei ca. 50°C durchgeführt werden. Der zweite Schritt der Reaktion kann mit $NaBH_4$ in 2-Propanol erfolgen.

Im nachfolgenden wird die Synthese eines erfindungsgemäßen dreikettigen Lipohexapeptids beschrieben.

**Beispiel 17**

**N(α-hexadecyl-β-stearoyloxy-arachidoyl)-alanin-tert.-butylester**

Stearinsäure (0,7 g; 2,46 mMol) wird in THF/$CHCl_3$ 1:9 gelöst und DCC (0,25 g; 1.23 mMol) dazugegeben. Nachdem Dicyclohexylharnstoff ausgefallen ist, wird in $CHCl_3$ gelöstes N-(α-hexadecyl-β-hydroxy-arachidoyl)-alanin-tert.-butylester (0,84 g; 1.23 mMol), sowie N,N-Dimethylaminopyridin (15 mg; 0.12 mMol) und N-Methylmorpholin (130 µl; 1.23 mMol) zugefügt. Nach 24 Stunden wird das Lösungsmittel am Rotationsverdampfer entfernt und der Rückstand in Essigester digeriert. Es wird von nicht löslichem Dicyclohexylharnstoff abfiltriert und das Filtrat eingeengt. Der Rückstand wird in wenig Aceton gelöst und bis zur Trübung Methanol zugegeben. Es wird im Eisbad gefällt, das Produkt wird abgesaugt und aus tert.-Butanol lyophilisiert.

Ausbeute: 0,7 g (60 %), Schmp. 71°C, Molmasse 947 (FD-MS)

**N-(α-hexadecyl-β-stearoyloxy-arachidoyl)-alanin**

N-(α-hexadecyl-β-stearoyloxy-arachidoyl)-alanin-tert.-butylester (0,33 g; 0.34 mMol) wird in wenig Methylenchlorid gelöst und 5 ml Trifluoressigsäure zugegeben. Nach 1 Stunde wird das Lösungsmittel am Rotationsverdampfer entfernt. Der Rückstand wird in Chloroform gelöst und Ether bis zur Fällung zugetropft. Es wird abgesaugt, mit Ether gewaschen und aus tert.-Butanol lyophilisiert.

Ausbeute: 0,26 g (87 %), Schmp. 72°C.

$C_{57}H_{111}NO_5$ (890.5)

ber.: C 76.88 H 12.56 N 1.57

gef.: C 76.87 H 12.72 N 1.30

**N-(α-hexadecyl-β-stearoyloxy-arachidoyl)-alanin-N-hydroxysuccinimid-ester**

N-(α-hexadecyl-β-stearoyloxy-arachidoyl)-alanin (0,2 g; 0.2 mMol), N-hydroxysuccinimid (52 mg; 0.44 mMol) und DCC (41 mg; 0.2 mMol) in DMF werden 4 Stunden bei Raumtemperatur gerührt. Anschließend wird das Lösungsmittel im Vakuum entfernt, 30 ml $CHCl_3$/MeOH 1:1 zugegeben und im Eisbad gefällt. Das Produkt wird aus tert.-Butanol lyophilisiert.

Ausbeute: 0,175 g (80 %), $R_f$ (Essigester, wassergesättigt) = 0.83, Schmp. 75°C.

$C_{61}H_{114}N_2O_7$ (987.6)

ber.: C 74.19 H 11.63 N 2.84

gef.: C 74.12 H 11.40 N 2.19

**N-[α-hexadecyl-β-stearoyloxy-arachidoyl]-alaninyl-O-tert.-butyl-seryl-N$_\epsilon$-tert.-butyloxycarbonyl-lysyl-N$_\epsilon$-tert.-butyloxycarbonyl-lysyl-N$_\epsilon$-tert.-butyloxycarbonyl-lysyl-N$_\epsilon$-tert-.butyloxycarbonyl-lysin-tert.-butylester ($C_{36}$-Myc(Ste)-Ala-Ser(tBu)-[Lys(Boc)]$_4$-OtBu)**

Eine Lösung von N-(α-hexadecyl-β-stearoyloxy-arachidoyl)-alanin-N-hydroxy-succinimid-ester (25 mg; 25 µMol) und H-Ser(tBu)-(Lys(Boc))$_4$-OtBu (30 mg; 27 µMol) in DMF wird nach Zugabe eines Tropfens N-Methylmorpholin 24 Stunden bei Raumtemperatur gerührt. Das Lösungsmittel wird anschließend abgedampft und der Rückstand an (R)Sephadex LH-20 ($CHCl_3$/MeOH 1:1, Säule 50x1.5 cm) chromatographiert. Die Lipopeptidfraktionen werden vereint, das Lösungsmittel wird abgedampft und das Lipopeptid aus tert.-Butanol lyophilisiert. Es wird so ein in Chloroform lösliches, farbloses Pulver erhalten.

Ausbeute: 22 mg (42 %).

$C_{116}H_{215}N_{11}O_{21}$ (2100.0); FD-MS: (M + Na)-2027

**N-(α-hexadecyl-β-stearoyloxy-arachidoyl)-alaninyl-seryl-lysyl-lysyl-lysyl-lysin ($C_{36}$-Myc(Ste)-Ala-Ser-(Lys)$_4$-OH)**

$C_{36}$-Myc(Ste)-Ala-Ser (tBu)-[Lys(Boc)]$_4$-OtBu (22 mg; 10 µMol) wird mit wasserfreier Trifluoressigsäure 1 Stunde behandelt. Durch mehrfaches Abziehen des Lösungsmittels und Verdünnen mit Chloroform wird Trifluoressigsäure entfernt. Das ungeschützte Lipopeptid wird in tert.-Butanol aufgenommen und lyophilisiert.

Ausbeute: 16 mg (87 %).

Aminosäureanalyse: Ala 0.23 (1)*, Ser 1.11 (1), Lys 4 (4),

Recovery 87 %. FD-MS:M + H 1490 (ber. 1489.3).

Fig. 1 zeigt die Dosis-Wirkungskurve für den $^3$H-Thymidin-Einbau in die DNA von Balb/c Mäusen, der durch $C_{36}$-Myc(Ste)-Ala-Ser-(Lys)$_4$-OH (gestrichelte Kurve) induziert wird im Vergleich mit dem starken Mitogen [2,3-bis(palmitoyloxy)-(2RS)-propyl]-N-palmitoyl-cysteinyl-seryl-seryl-asparaginyl-alanin (durchgezogene Kurve) (K.H. Wiesmüller, W.G. Bessler, G. Jung, Hoppe-Seylers Z. Physiol. Chem. 364 - (1983), 593-606. Ordinate: eingebautes [$^3$H] Thymidin in [cpm x 1000]; Abszisse: Mitogen Konzentration in [µg/ml.

## Ansprüche

1. Verfahren zur Herstellung von lipophilen Aminosäurederivaten, gekennzeichnet durch Umsetzen eines Aldoketen-Dimeren der allgemeinen Formel (I)

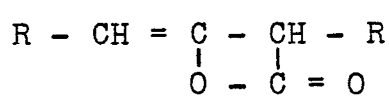

(I)

wobei R = Alkyl mit mehr als 6 C;

mit einem Aminosäureester mit freiem N Terminus zu einem α-Alkyl-β-ketoacyl-aminosäurederivat der allgemeinen Formel (II):

$$R - CH_2 - CO - \underset{R}{\underset{|}{CH^*}} - CO - NH - \underset{R'}{\underset{|}{CH^*}} - COX \qquad (II)$$

wobei R′ = Seitenkette einer (Protein-)Aminosäure oder H und X = Schutzgruppe für eine Carboxylsäuregruppe, wie $OBu^t$, OMe, OBzl oder dergleichen, oder eine geschützte Aminosäure oder Peptid, ist.

2. Verfahren nach Anspruch 1, dadurch gekennzeichnet, daß das Aldoketen-Dimere (I) ein Fettsäure-Aldoketen-Dimeres ist.

3. Verfahren nach einem der Ansprüche 1 oder 2, gekennzeichnet durch Umsetzen des Aldoketen-Dimeren mit dem Aminosäureester in Gegenwart einer katalytischen Menge eines Dialkylaminopyridins mit Niederalkylresten ($C_1$-$C_6$), bevorzugt von Dimethylaminopyridin.

4. Verfahren nach einem der Ansprüche 1 bis 3, dadurch gekennzeichnet, daß die Umsetzung in einem organischen Lösungsmittel, bevorzugt trockenem Pyridin, erfolgt.

5. Verfahren nach einem der vorangehenden Ansprüche, dadurch gekennzeichnet, daß die Umsetzung bei einer Temperatur zwischen 0 und 100°C, bevorzugt zwischen 25 und 80°C und ganz besonders bevorzugt zwischen 40 und 60°C erfolgt.

6. Verfahren nach einem der vorangehenden Ansprüche, ferner gekennzeichnet durch Reduktion der N-α-Alkyl-β-keto-acyl-Verbindung (II), bevorzugt mit einem Borhydrid in an sich bekannter Weise zu N-(α-Alkyl-β-hydroxy-acyl-Verbindungen der allgemeinen Formel (III):

$$R - CH_2 - \underset{OH}{\underset{|}{CH}} - CHR^* - CO - NH - \underset{R'}{\underset{|}{CH^*}} - CO - X \qquad (III)$$

7. Verfahren gemäß Anspruch 6, ferner gekennzeichnet durch Veresterung des Reaktionsproduktes (III) in an sich bekannter Weise, bevorzugt durch Umsetzung mit einem Säureanhydrid unter Erhalt von Estern der nachfolgenden allgemeinen Formel (IV):

$$R - CH_2 - \underset{\begin{array}{c}|\\ O\\ |\\ R'' - C = O\end{array}}{CH} - CHR - CO - NH - CHR' - CO - X \qquad (IV)$$

8. Verfahren nach einem der vorangehenden Ansprüche, gekennzeichnet durch Abspaltung der Carboxyl-Schutzgruppe, bevorzugt mit $CF_3COOH$ unter Erhalt der freien Carboxylgruppe(n).

9. Verfahren nach Anspruch 6, gekennzeichnet durch Behandeln der α-Alkyl-β-hydroxy-Verbindung mit Acetanhydrid und $KHSO_4$ unter Erhalt von Verbindungen der allgemeinen Formel (V):

$$R - CH_2 - CH = \underset{R}{\underset{|}{C}} - CO - NH - \underset{R'}{\underset{|}{CH}} - CO - X \qquad (V)$$

10. Lipophile Aminosäurederivate der allgemeinen Formel (V):

$$R - CH_2 - CH = \underset{R}{\underset{|}{C}} - CO - NH - \underset{R'}{\underset{|}{CH}} - CO - X \qquad (V)$$

wobei R = Alkyl mit mehr als 6 C; R′ = Seitenkette einer (Protein-)Aminosäure oder H und X = Schutzgruppe für Carboxylsäuregruppe, eine geschützte Aminosäure oder Peptid oder -OH ist, erhältlich nach dem Verfahren des Anspruches 9.

11. Lipophile Aminosäurederivate der allgemeinen Formel (IV):

$$R - CH_2 - \underset{\begin{array}{c}|\\ O\\ |\\ R'' - C = O\end{array}}{CH} - CHR - CO - NH - CHR' - CO - X \qquad (IV)$$

wobei R = Alkyl mit mehr als 6 C; R′ = Seitenkette einer (Protein-)Aminosäure oder H ist, R″ = beliebiger Alkylrest und X = Schutzgruppe für Carboxylsäuregruppe, eine geschützte Aminosäure oder Peptid oder -OH ist, erhältlich nach dem Verfahren des Patentanspruchs 8.

12. Verwendung von Verbindungen der Formel II, III, IV oder V gemäß einem oder mehreren der Ansprüche 1 - 11 als mitogene Substanz und/oder Adjuvans.

**Patentansprüche für die folgenden Vertragsstaaten: ES, GR**

1. Verfahren zur Herstellung von lipophilen Aminosäurederivaten, gekennzeichnet durch Umsetzen eines Aldoketen-Dimeren der allgemeinen Formel (I)

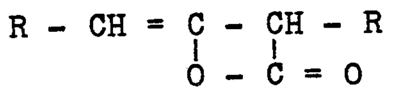

(I)

wobei R = Alkyl mit mehr als 6 C;
mit einem Aminosäureester mit freiem N-Terminus zu einem α-Alkyl-β-ketoacyl-aminosäurederivat der allgemeinen Formel (II):

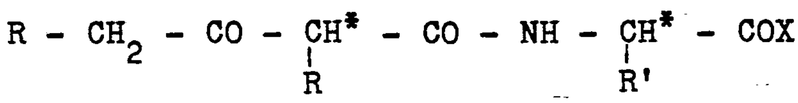

(II)

wobei R′ = Seitenkette einer (Protein-)Aminosäure oder H und X = Schutzgruppe für eine Carboxylsäuregruppe, wie $OBu^t$, OMe, OBzl oder dergleichen, oder eine geschützte Aminosäure oder Peptid, ist.

2. Verfahren nach Anspruch 1, dadurch gekennzeichnet, daß das Aldoketen-Dimere (I) ein Fettsäure-Aldoketen-Dimeres ist.

3. Verfahren nach einem der Ansprüche 1 oder 2, gekennzeichnet durch Umsetzen des Aldoketen-Dimeren mit dem Aminosäureester in Gegenwart einer katalytischen Menge eines Dialkylaminopyridins mit Niederalkylresten ($C_1$-$C_6$), bevorzugt von Dimethylaminopyridin.

4. Verfahren nach einem der Ansprüche 1 bis 3, dadurch gekennzeichnet, daß die Umsetzung in einem organischen Lösungsmittel, bevorzugt trockenem Pyridin, erfolgt.

5. Verfahren nach einem der vorangehenden Ansprüche, dadurch gekennzeichnet, daß die Umsetzung bei einer Temperatur zwischen 0 und 100°C, bevorzugt zwischen 25 und 80°C und ganz besonders bevorzugt zwischen 40 und 60°C erfolgt.

6. Verfahren nach einem der vorangehenden Ansprüche, ferner gekennzeichnet durch Reduktion der N-α-Alkyl-β-keto-acyl-Verbindung (II), bevorzugt mit einem Borhydrid in an sich bekannter Weise zu N-(α-Alkyl-β-hydroxy-acyl-Verbindungen der allgemeinen Formel (III):

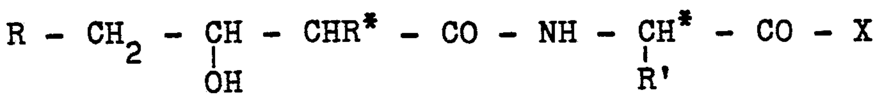

(III)

7. Verfahren gemäß Anspruch 6, ferner gekennzeichnet durch Veresterung des Reaktionsproduktes (III) in an sich bekannter Weise, bevorzugt durch Umsetzung mit einem Säureanhydrid unter Erhalt von Estern der nachfolgenden allgemeinen Formel (IV):

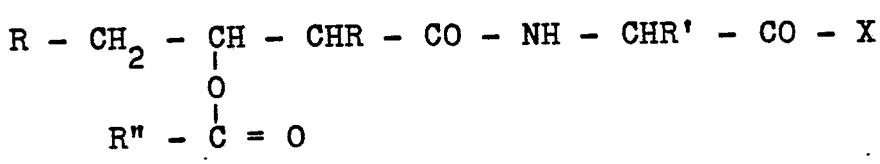

(IV)

8. Verfahren nach einem der vorangehenden Ansprüche, gekennzeichnet durch Abspaltung der Carboxyl-Schutzgruppe, bevorzugt mit $CF_3COOH$ unter Erhalt der freien Carboxylgruppe(n).

9. Verfahren nach Anspruch 6, gekennzeichnet durch Behandeln der α-Alkyl-β-hydroxy-Verbindung mit Acetanhydrid und $KHSO_4$ unter Erhalt von Verbindung der allgemeinen Formel (V):

$$R - CH_2 - CH = \underset{R}{\underset{|}{C}} - CO - NH - \underset{R'}{\underset{|}{CH}} - CO - X \qquad (V)$$

10. Verwendung von Verbindungen der Formel II, III, IV oder V gemäß einem oder mehreren der Ansprüche 1 - 11 als mitogene Substanz und/oder Adjuvans.

Fig. 1

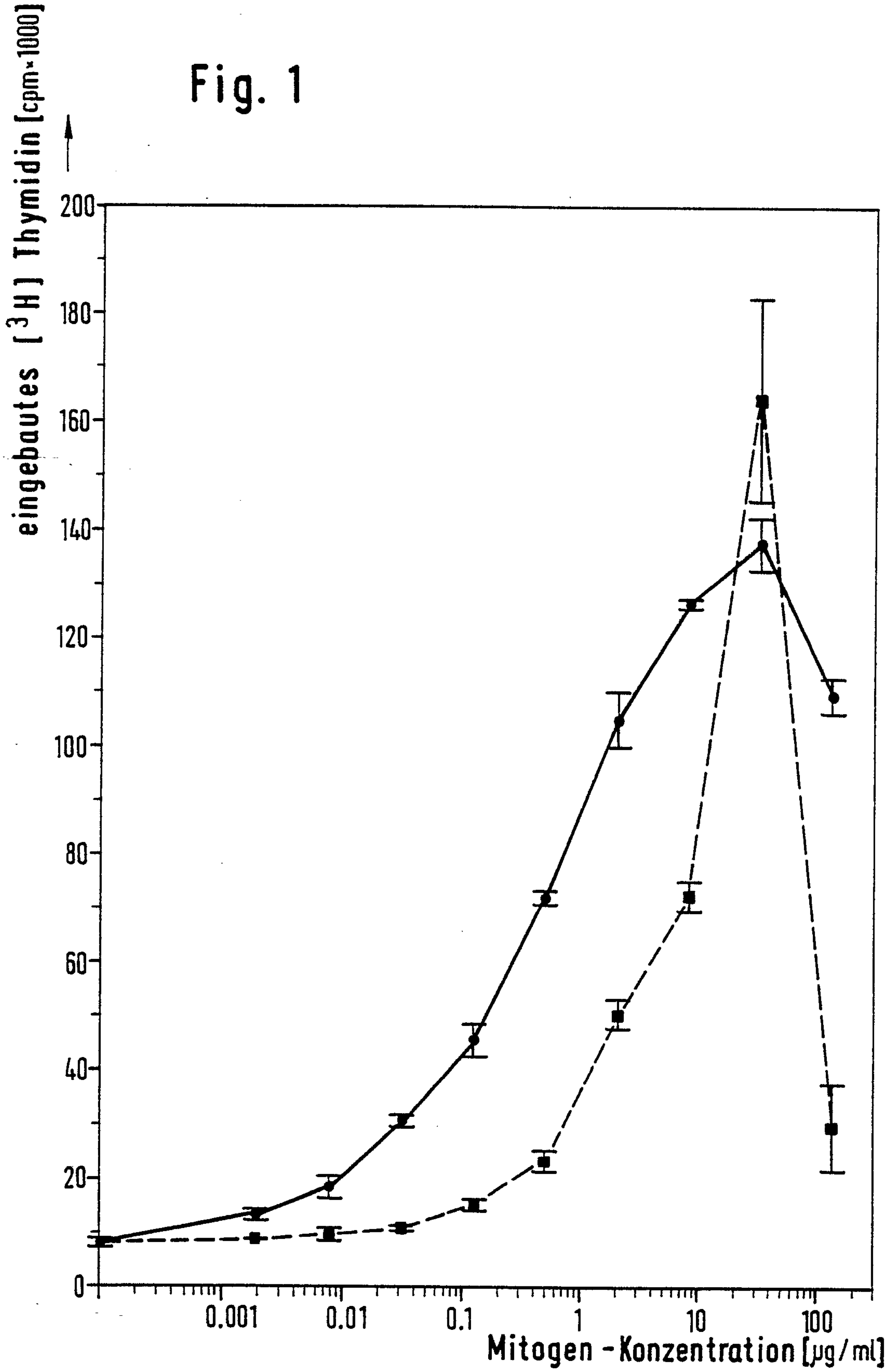
eingebautes [3H] Thymidin [cpm×1000]
200
180
160
140
120
100
80
60
40
20
0
0.001
0.01
0.1
1
10
100
Mitogen - Konzentration [µg/ml]